# EUROPEAN PATENT SPECIFICATION

(11) **EP 1 089 679 B1**
(45) Date of publication and mention of the grant of the patent: **07.09.2005**
(21) Application number: 99930719.2
(22) Date of filing: 25.06.1999
(51) Int. Cl.: A61F 5/41, A61K 9/00, A61P 15/10

(54) **A DEVICE AND METHOD FOR THE TREATMENT OF ERECTILE DYSFUNCTION**
VORRICHTUNG UND VERFAHREN ZUR BEHANDLUNG VON EREKTIONSSTÖRUNGEN
DISPOSITIF ET METHODE POUR TRAITER LA DYSERECTION

(30) Priority: 25.06.1998 US 90674 P
(43) Date of publication of application: 11.04.2001
(73) Proprietor: Lavipharm Laboratories, Inc., Piscataway, NJ 08854 (US)
(72) Inventor: FOTINOS, Spiros, GR-106 72 Athens (GR)
(74) Representative: Breese, Pierre
(86) International application number: PCT/US1999/014410
(87) International publication number: WO 1999/066870

(56) References cited:
- EP-A- 0 266 968
- FR-A- 2 748 658
- US-A- 4 829 991
- US-A- 4 867 982
- US-A- 5 302 395
- US-A- 5 333 621
- US-A- 5 741 511

## Description

### Technical Field

Delivery devices, methods of manufacture and uses for noninvasive treatment of erectile dysfunction are provided.

### Background Art

Erectile dysfunction is considered a common disorder affecting approximately 10% of men of any age. The incidence of this dysfunction, however, increases dramatically with age. By definition, erectile dysfunction is the inability to attain and maintain the erection of the penis sufficient time to permit satisfactory sexual intercourse. The cause of erectile dysfunction may be of psychogenic, organic, iatrogenic, or hormonal origin. Psychogenic based erectile dysfunction may occur in patients under age 35 due to psychological disorders such as depression, anxiety, stress, performance anxiety etc. Organic based dysfunction may occur in older patients due to a vascular, cavernosal or neurological disease such as atherosclerosis, hypertension, smoking, diabetes, spinal injury, multiple sclerosis etc. Iatrogenic based dysfunction mainly appcars after the administration of drugs or the performance of medical/surgical procedures. It has been reported that over 130 drugs can cause erectile dysfunction as a side effect, including for example such drugs as antihypertensives (e.g. β-blockers and diuretics) and hypoglycemics. Hormonal based dysfunction mainly occurs due to changes in testosterone levels or other hormones or chemical messengers.

The cause of impotence commonly determines the treatment of penile erectile dysfunction, with the ultimate goal of recovering sexual activity. Treatment may involve procedures such as psychotherapy, vascular surgery, or prosthetic surgery. Alternatively, therapeutic agents may be selected for pharmacotherapy which can be administered orally, topically, intra-urethrally or injected intracavemously (EP 702555; EP 249194; EP 459377; U.S. patents 5,242,391; 5,474,535; 5,492,911; 5,576,290;5; 658,936; 5,698,589; and 5,842,039). In certain circumstances, specific devices may be used in treatment such as a vacuum cylinder device. The cause of the dysfunction may determine the therapeutic approach of choice.

Where therapeutic agents are used, different routes of administration of the agent to the subject are associated with certain disadvantages. For example, the intra-urethral route of administration may cause side effects for both patient and partner because a portion of the administered drug remains in the urethra and, thus, can be transmitted to the partner during intercourse. The self-injection route during intracaverneous administration has disadvantages associated with lack of spontaneity and unpleasant and sometimes pathological side effects and suffers from a high drop-out rate of the user population. Although oral administration is a convenient and attractive approach of therapy, this approach is open to abuse by patients without erectile dysfunction, but who would try an oral formulation for improving performance. The topical route relying on a gel, cream, or ointment lacks dose precision at the site of application, namely the penis skin. Transdermal delivery methods have been utilized for the systemic treatment of certain disorders as in U.S. patents 5,5152,997; 4,812,313; 4,954,344; and 5,302,395. Several trials to optimize drug delivery to the penis by the transdermal route leading to successful sexual intercourse have resulted in developing a variety of transdermal drug delivery systems. There are however problems associated with these systems relating to the change in the surface texture of the penis and the implications of friction of penis against vagina.

Where attempts have been made to employ transdermal delivery devices for the treatment of erectile dysfunction, a rather complicated vasodilator delivery system has been developed (U.S. 5,333,621) which is complex in design and inconvenient to use. The system consists of a three or two adhesive layers, which are adhered inside a condom. Upon use, the three layer patch is adhered to the penis skin after applying pressure to the condom.

Another transdermal delivery device for the treatment of male impotence is described in U.S. 5,741,511. The device is in the form of a cylinder matrix type or a multi-reservoir patch applied to the glans penis. Even in this case, to obtain the maximum of the desired effect the patch has to be accompanied by a support and rings attached to the penis. After achieving enough erection, the patch and support are removed while the rings remain during the intercourse. This device is unpleasant and inconvenient for both the patient and his partner.

Still another transdermal delivery device (patch) containing prostaglandin for the treatment of any pathological condition (e.g. peripheral arterial occlusive disease) is described in U.S. 5,480,648. The patch described in 648 consists of a pressure sensitive adhesive containing the active and other additives, laminated onto a backing film. However, the drug release profile of this patch disclosed long term delivery which is unsuited for the treatment of erectile dysfunction because an effective dose of an active agent is required over a short period of time prior to intercourse so as to provide a positive response (rigid tumescence of penis).

Therefore, there is a need to develop a system and use for the treatment of erectile dysfunction that is simple, safe, convenient and painless to use, but not open to abuse and that delivers an effective "therapeutic dose" to the penis over a short period of time and is applied directly to the penis surface without using any additional support, and eventually enabling the patient to achieve normal sexual activity.

### Summary of the Invention

Accordingly, the invention in a first embodiment is a delivery device for treatment of erectile dysfunction in a patient, comprising a disk formed from a filmogenic polymer, and having an effective dose of a therapeutic agent suitable for reversing erectile dysfunction. This embodiment comprises further at least one additive contained within the disk, wherein the at least one additive is selected from the group consisting of a stabilizer, a solubilizer, an enhancer and a plasticizer. The therapeutic agent is in one embodiment a prostaglandin, preferably prostaglandin E1. In another embodiment, the therapeutic agent is selected from the group consisting of: a vasodilator, a smooth muscle relaxant, an anti-depressant, a parasympathetic stimulator, a renin-angiotensin system inhibitor, a local anesthetic, an α-blocker, and a calcium channel blocker. Further, the device can comprise further at least an additional therapeutic agent. For example, the at least one additional therapeutic agent can be selected from the group consisting of: a prostaglandin, a testosterone, a yohimbine, a pentoxifylline, a trazodone, an apomorphine, a phentolamine, a sildenafil, a minoxidil, a misoprostol, a papaverine, a nitroglycerin, a phentolamine, a moxisylyte, a linsidomine, a linear peptide, a cyclic peptide, and a pyridylguanidine compound.

In the embodiment of the delivery device, the enhancer is at least one selected from the group consisting of a glycolipid, a non-esterified fatty acid, an aliphatic alcohol, a fatty acid ester of an aliphatic alcohol, a cyclohexanol, a cyclohexanol derivative, a fatty acid ester of glycerol, a glycol, an aliphatic aiconol ether of a glycol, and a surfactant. In a preferred embodiment of the device, the filmogenic polymer is polyvinyl pyrrolidone, the therapeutic agent is prostaglandin E1, the enhancer is Eutanol G16S, and the plasticizer is PEG 400. Further, the filmogenic material is present in an amount of 5 to 100%, the therapeutic agent is present in an amount of 0.1 to 20% w/w, the enhancer is present in an amount of 0.01 to 15%, and the plasticizer is present in an amount of I to 70%, each on a weight basis. In another embodiment of the delivery device, the filmogenic material is present in an amount of 5 to 50%, the therapeutic agent present in an amount of 1 to 10%, the permeation enhancer present in an amount of 1 to 10%, and the plasticizer present in an amount of 3 to 50%. A preferred embodiment has polyvinyl pyrrolidone present in an amount that is 40 to 45%, having prostaglandin E1 present in an amount that is 5 to 10%, having Eutanol G16S present in an amount that is 1 to 4%, and having PEG 400 present in an amount that is 40 to 50%. When PEG 400 is present in an amount less than 30% on a dry weight basis, a rigid disk is formed.

In another aspect of the delivery device delivery is transdermal. In a different embodiment delivery is transmucosal. The effective dose is released into the subject within one hour.

In another embodiment, the invention is a use for treating erectile dysfunction, comprising: selecting a disk formed from a filmogenic polymer and comprising one or more therapeutic agents selected from the group consisting of a vasodilator, a smooth muscle relaxant, an anti-depressant, a parasympathetic stimulator, a renin-angiotensin system inhibitor, a local anesthetic, an α-blocker, and a calcium channel blocker, and delivering the therapeutic agent to the penile surface over an effective period of time. In forming the disk, the therapeutic agent is selected from the group consisting of a prostaglandin, a testosterone, a yohimbine, a pentoxifylline, a trazodone, an apomorphine, a phentolamine, a sildenafil, a minoxidil, a misoprostol, a papaverine, a nitroglycerin, a phentolamine, a moxisylyte, a linsidomine, a linear peptide, a cyclic peptide, and a pyridylguanidine compound. The therapeutic agent is present in a range of 0.1-15%, on a dry weight basis. In one aspect of this embodiment, the disk further comprises adding a plasticizer, for example, the plasticizer is present in an amount that is 30 to 60% on a dry weight basis, and delivering the therapeutic agent to the penile surface does not require pre-wetting. In another aspect of this embodiment, the plasticizer is present in an amount that is less than 30% on a dry weight basis, and delivering the therapeutic agent to the penile surface has the additional step of pre-wetting the surface. Preferably, the plasticizer is a polyethylene glycol (PEG), even more preferably, the PEG is PEG 400. In this embodiment, the filmogenic polymer can be a synthetic polymer, for example, the synthetic polymer is polyvinyl pyrrolidone. Further, the filmogenic polymer can be a plant protein, for example, a prolamine, preferably a gliadin or a mixture of gliadins. In these embodiments, the effective period of time is 5-100 minutes, for example, the effective period of time is 30-60 minutes. In these embodiments, the penile surface is selected from the group consisting of the shaft and the glans.

Yet another embodiment of the invention is method of preparation of a flexible disk for use in the treatment of erectile dysfunction, comprising: preparing a composition having prostaglandin E1, Eutanol G16S. PVP, and PEG 400; and forming the composition to have a backing and a release layer. Preferably, the method of preparing the composition includes adding prostaglandin E1 in an amount that is 5 to 10%, adding Eutanol G16S in an amount that is 1 to 5%, adding polyvinyl alcohol pyrrolidone in an amount that is 40 to 50%, and adding PEG 400 in an amount that is 40 to 50%, each on a dry weight basis.

### Brief Description of the Figure

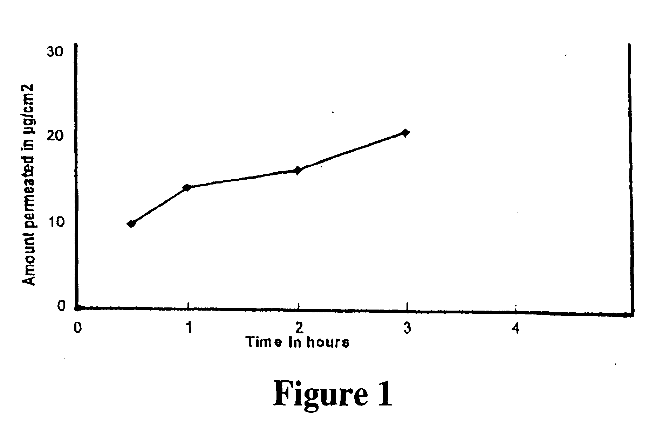

Fig. 1 is a graph showing cumulative permeation of prostaglandin per unit area of stratum corneum as a function of time, in response to administration of the formulation of Example 9.

### Description of the Specific Embodiments

In accordance with an embodiment of the invention, a biodegradable system in the form of a flexible disk is provided which may be applied directly to the surface of the penis, for example, the penis shaft or the glans. The flexible disk may include an effective dose of a therapeutic agent for the treatment of erectile dysfunction and further may include a carrier for the delivery of the therapeutic agent. In a preferred embodiment, the disk is biodegradable. The flexible disk includes a backing material and a release liner, which is removed prior to application to the penile skin. Formulations of Examples 1-6 and 9 are used to provide a flexible device which is manufactured with a backing and a release liner. Flexibility is contributed by the presence of a plasticizer, for example PEG 400, as a substantial percentage of the formulation of these Examples, for example, PEG 400 is present in an amount that is 30-60% on a dry weight basis, preferably 40-50%, even more preferably 44-48% of the formulation on a dry weight basis.

According to an embodiment of the present invention, the disk can be made of materials known in the art to possess filmogenic properties. The filmogenic materials can preferably be any of synthetic origin, such as polyvinyl pyrrolidone (PVP) or a PVP derivative or another pressure-sensitive adhesive: or the materials can be of semi-synthetic origin such as any of different cellulose derivatives. The filmogenic materials can be of natural origin, such as a polymer from an animal, for example chitin or chitosan from a crustacean exoskeleton, or from a plant, such as a gum, for example, xanthin gum, karaya gum, or a plant resin, for example mastic (for example from *Pistachia lentiscus* var. Chios), or a plant protein such as from wheat or corn, such as a prolamine protein. The prolamine protein can be a zein from maise, a hordein from barley, or a gliadin from wheat. The filmogenic plant protein polymer can be a mixture of proteins, for example, a mixture of wheat gliadins.

In one embodiment, the disk is flexible and contains PVP at a concentration of less than 50%, in which embodiment the PVP is a pressure-sensitive adhesive. In another embodiment, the disk is rigid and requires application to a wet surface, in which embodiment PVP at a higher concentration, for example 90-95%, is a filmogenic material.

In an embodiment of the invention, the disk is rigid and does not require supporting means distinct from the disk, i.e., does not require a backing or a release liner. An embodiment of the invention provides a method of treating male impotence, the method including applying the drug-containing disk to the surface of the penis with pre-wetting the arca with a liquid, for example, with water, or an aqueous solution, or a glycol, or any liquid capable both of wetting the disk and of not causing discomfort to the user. A formulation of this embodiment is shown in Example 7, in which the filmogenic synthetic polymer PVP is present in a high amount (91 %), and PEG is present as a minor component (3% on a dry weight basis), and in Example 8, in which the filmogenic natural plant protein gliadin is present in a high amount (93.30%), and polysorbate monooleate is present at a low amount (5.00%).

Other additives can be incorporated into the disk to optimize its physicochemical and mechanical properties. The additives may include stabilizers, solubilizers, enhancers, and plasticizers. Permeation enhancers for transmucosal (via the glans) or for transdermal (via the shaft) delivery can be employed for improving the absorption of therapeutic agents through the penis surface to the corporal cavernosa. These may include but are not limited to: glycolipids, fatty acids, aliphatic alcohols, cyclohexanols and their derivatives, esters of glycerol with fatty acids, glycols and ethers of glycols with aliphatic alcohols, surfactants and other compounds known by one of ordinary skill in the art of transdermal and transmucosal drug delivery. Examples of skin permeation enhancers for use in the invention include a glycolipid mixture of plant origin, linoleic acid, polyethylene glycol ether of lauryl alcohol (Lipocol-12), L-menthol, hexyldecyl stearate (Eutanol G16S), polysorbate monooleate, isopropyl myristate, and glycerol monooleate.

The term "transdermal" as used herein and in the claims means a route of delivery of one or more therapeutic agents across the epithelial layer of a patient, including across the stratum corncum comprising dead differentiated epithelial cells that have produced a dry keratinized layer. The term "transmucosal" as used herein shall mean a route of delivery of one or more therapeutic agents across a mucosal epithelial layer. A mucosal epithelial layer of is found in an oral (buccal) cavity and in the vaginal cavity of a patient, and in an uncircumsized male, beneath the foreskin of the penis on the glans. Transmucosal and transdermal drug deliverery systems are described in "Transdermal and Topical Drug Delivery Systems." Ed. Ghosh. T. et al. (Buffalo Grove, IL: Interpharm Press, Inc., 1997), which describes the higher permeability of mucosal tissue.

An embodiment of the invention which is a rigid disk is suitable for application to the penis shaft or to the glans, for transdermal or transmucosal delivery, respectively, of a therapeutic agent or combination of agents. A user of the rigid disk can in certain circumstances apply the device to the glans without requiring a pre-wetting step. Further, as the higher permeability of mucosal tissue results in the transmucosal route of delivery having more efficient and rapid uptake of a therapeutic agent, a formulation specific for transmucosal delivery can contain decreased amounts of each of the therapeutic agent and the permeation enhancer, compared to a formulation specific for transdermal delivery via the shaft. Alternatively, a user of the rigid disk can shorten the period of application of the disk to the surface of the penis if a transmucosal route, rather than a transdermal route of delivery is chosen, so that a single formulation for both routes can be employed.

Therapeutic agents incorporated in a disk of the invention can include any drug known in the art for the treatment of erectile dysfunction, alone or in combination, preferably drugs that could be administered topically through the skin of the shaft and/or the glans of the penis, such as vasodilators, smooth muscle relaxants, local anesthetics, α-blockers, and calcium channel blockers.

A therapeutic agent present in the device formulations can include a hormone, for example, a steroid hormone such as testosterone, a peptide hormone, an amine hormone, and a hormone-like eicosanoid such as a prostaglandin, a leukotriene, and a thromboxane. In a preferred embodiment, an effective dose of a peripheral vasodilator such as an eicosanoid, preferably a prostaglandin, more preferably, prostaglandin E1 alone or in combination with other therapeutic agents, may he used. In another preferred embodiment, a combination of prostaglandin E1 with a low concentration of a second vasodilator, exemplified by a venous dilator such as nitroglycerin, may be used.

The term "prostaglandin" refers to a family of compounds originally discovered in seminal fluid and found to cause vasodilation, and contraction or relaxation of uterine smooth muscle. The prostaglandins, leukotrienes, and related compounds are called eicosanoids because they are synthesized by microsomal enzymes from 20-carbon essential fatty acids, e.g., arachidonic acid (Hardman, J. 1996, in Goodman and Gilmans's: The Pharmacological Basis of Therapeutics, Ch. 26. 9^{th} Ed.. McGraw Hill).

In certain embodiments of the invention, additional therapeutic agents may be included in the disk either singly, or in selected combination with any one or more of the aforementioned or following therapeutic agents; testosterone, yohimbine, pentoxifylline (vasodilator), trazodone (antidepressant,) apomorphine (parasympathetic stimulator) phentolamine (vasodilator), sildenafil and other pyrozolopyrimidone derivatives. Other agents include minoxidil, misoprostol, papaverine, nitroglycerin, phentolamine, moxisylyte, linsidomine, linear peptides, cyclic peptides, pyridylguanidine compounds, and renin-angiotensin system inhibitors. These agents may be incorporated into a disk at an effective dose to correct erectile dysfunction of the penis. Using a transdermal or a transmucosal device that is an embodiment of the invention, the disk can accommodate a drug load that is ten- to twenty-fold greater than that used for intracavernosal injection as a treatment for erectile dysfunction.

According to an embodiment of the invention, an effective amount of the active agent is released in a time period that is desirable to obtain a positive response (rigid tumescence of penis) for a satisfactory sexual intercourse, for example, 30 to 60 min after application.

According to a preferred embodiment, the invention provides a biodegradable disk comprising, related to the total weight of the carrier and other ingredients: one or more therapeutic agents for the treatment of erectile dysfunction in an amount of 0.1 to 20% w/w, preferably in an amount of 0.1 to 15% w/w on a dry weight basis, more preferably in an amount of 1 to 10% w/w: one or more skin permeation enhancers in an amount of 0.01 to 15% w/w, preferably of 1 to 10% w/w; one or more filmogenic materials in an amount of 5 to 100% w/w, for example, 5 to 50%, 20-50%, 40-60%, and 70-95% w/w; and one or more plasticizers in an amount of 1 to 70% w/w, preferably 3 to 50% w/w.

According to an embodiment of the invention that is a flexible device and that does not require a pre-wetting step, the disk may be placed during manufacturing between two protective layers, namely backing film and release liner, wherein the latter is removed prior to use. Both backing film and release liner can be made of any suitable material known in the art of transdermal drug delivery. Further, the pressure-sensitive adhesive component in the formulation of the flexible disk may be reduced to obtain comfortable application to and removal from the glans, to obtain rapid transmucosal delivery of the therapeutic agents.

An embodiment of the invention is a delivery device for a therapeutic agent for reversing erectile dysfunction, comprising: a flexible disk, the disk being formed from a filmogenic polymer; and containing an effective dose of a therapeutic agent suitable for reversing erectile dysfunction. Delivery in one embodiment can be by a transdermal route, and in another embodiment by a transmucosal route. The device can comprise further at least one additive contained within the disk, wherein the at least one additive is selected from the group consisting of a stabilizer, a solubilizer, an enhancer and a plasticizer. The enhancer is selected from the group consisting of a glycolipid, a fatty acid for example a non-esterified fatty acid, a fatty acid ester of an aliphatic alcohol, an alcohol, a cyclohexanol, an ester of glycerol with one or more fatty acids, a glycols and an ether of a glycol with aliphatic alcohols, a surfactant, and other compounds known in the art to be used in the present invention. The therapeutic agent is selected from the group consisting of: a vasodilator, a smooth muscle relaxant, a local anesthetic, an α-blocker, and a calcium channel blocker. With this device, the effective dose is released into the subject within one hour.

Another embodiment of the invention is a use for treating erectile dysfunction, comprising: selecting a disk formed from a filmogenic polymer and comprising an eicosanoid or a mixture of eicosanoids; and delivering the eicosanoid to the penile surface over an effective period of time. Accordingly, the eicosanoid in an embodiment of the invention is a prostaglandin, and the effective period of time is 5-100 minutes, preferably the effective period of time is 30-60 minutes.

### Fabrication of a device

A device of the composition in one embodiment can have a release liner layer placed on the composition layer on a surface opposite to that of the backing support. A device can be provided with a tab for convenient removal of the backing or the release liner or both, that has been sheltered from deposition of the composition. Similarly, a tab on the backing support is provided so the device can be grasped by the user for removal of the device from the skin.

The composition can be applied to a release liner or to a non-porous backing support by a standard coating process, for example, using a rotary doctor blade, slot die, a flat film die, or a laboratory coating unit with integrated dryer, for example, LTSV/LTF, W. Mathis AG, Niederhasli, Zurich, Switzerland CH-8155. A solution or dispersion of the composition in the solvent can be deposited as a film or matrix layer to a sheet of a release liner, dried to remove the solvent, and then covered by application of a sheet of backing support. Alternatively, the device can be produced as a layer of composition deposited on a backing support if the support is nonporous, then can be dried to remove the solvent.

An embodiment of a device comprises a backing support of a nonwoven, nonocclusive film of high moisture vapor transmission. The backing support can impart tensile strength so that the device can be handled easily, and applied and removed without leaving a portion of the composition or device remaining on the skin. The backing support can be nonporous, microporous, or macroporous, such as a woven or a nonwoven composition, and can be occlusive, scmiocclusive or nonocclusive to moisture. The nonwoven material is selected from the group consisting of polyethylene, polyolefin, polyurethane, polyester, polypropylene, nylon, cotton, rayon, polyvinyl chloride and a cellulosic fiber.

A release liner can be a material selected from the group consisting of paper, polyester, and polystyrene, and can be coated with a silicon or fluorocarbon based release coating. The release liner is removed from the device prior to applying the composition of the matrix layer to skin of a subject having erectile dysfunction. A release liner material can be MediRelease 2226, a polyester silicone release liner obtained from Bertek Medical Products. St. Albans. VT.

A method of fabrication of a device can be by application of a composition, for example, as in any of Examples 1-6 and Example 9, to deposit it on the surface of a large sheet of a release liner or a non-porous backing to a desired thickness, drying the matrix layer to the sheet, applying a backing or a release liner to the composition layer, and cutting the sheets in a pattern to produce a device which is an embodiment of the invention. Deposition of a solution or a suspension of the composition to a support substrate, can be, for example, to a release liner continuously supplied by a doctor roll apparatus, reverse roll, gravure, slot die or other suitable method to coat the adhesive onto the substrate.

The flexible device can have a thickness of one to 10 mil (a "mil" is 1/1000 of an inch), preferably a thickness or 2 to 5 mil, more preferably a thickness of 2.5 mil, even more preferably a thickness of 3.5 mil. The backing support can have a thickness of 0.5 to 10 mil, preferably a thickness of 3 to 5 mil. The release liner can have a thickness of 0.5 to 10 mil, preferably a thickness from 3 to 5 mil. The entire device can have a thickness of 3 to 50 mil, for example, can be 4, 8, 12, 16, 20, or 24 mil thick.

The laminated sheet can he cut into a plurality of devices using a stamp-type cutter, or by rotary die cutting, both techniques known to one of ordinary skill in the art. Embodiments of devices of the invention can be in the shape of rectangles ("strips"), circles, circular sectors, and other regular or irregular shapes such as fanciful shapes. A device can have an area of from 1-20 cm², for example, 1-5 cm², preferably 1-2 cm², for example, 1.1, 1.3, or 1.5 cm².

An embodiment of the device of the invention that is a rigid disk can be fabricated by forming a solution or suspension of the ingredients, and laminating the solution or suspension on a solid surface, for example, a surface of a polystyrene material. After drying to remove the solvent, the film is removed and cut into the desired shapes.

### Examples

### Examples 1-6. Flexible disks for treatment of erectile dysfunction.

A disk was manufactured by mixing solubilizer polyethylene glycol (PEG 400), a drug (prostaglandin E1) and a permeation enhancer (isopropyl myristate) under vigorous agitation to a mixture of polyvinyl pyrrolidone (PVP) in ethanol. The mixture thus obtained was coated on a siliconized polyester film and oven dried at 70°C for 15 minutes to form what is referred to here as "a system." The system was laminated on a polyethylene backing film, and patches of 1-20 cm², for example, 1-5 cm², preferably 1.3 cm², were formed. In this example, the drug load was 0.5 mg/cm². In vitro dissolution tests of the proposed formulations using US Pharmacopoeia No 23 methodology (apparatus 5) showed that the entire amount of the drug was dissolved within 30 min.

Compositions that are embodiments of the invention containing prostaglandin E1 (PGE1) in Examples 1-6 are shown in Table 1.

### Examples 7-8. Rigid disks for treatment of erectile dysfunction.

The systems having a formulation for Examples 7-8 shown in Table 1 are manufactured, and may be applied to a pre-wetted area of penile skin. In Example 7, the PVP content is 91.00 %, and that of PEG 400 is 3.00%, each on a dry weight basis. Alternatively, a formulation having the components of a Gliadin mixture (93.30 % dry weight) and Montane 80 VGA (5.00 dry weight) can be used in this embodiment of the invention, as shown in Example 8.

### Example 9. Determination of permeation of the components into human stratum corneum

To evaluate the permeability of the formulations disclosed by the present invention, a formulation containing prostaglandin E1.7%, Eutanol G16S. 2.5%, PVP, 43.73%, and PEG 400, 46.77%, each on a dry weight basis, was prepared by the method described above. The transdermal absorption (flux) of prostaglandin from the formulation was determined *in vitro* using human cadavar skin according to the procedure of Franz (J. Invest. Derm. 64, 190-105, 1975. Percutaneous absorption on the relevance of the *in vitro* data). The stratum corneum used in this example was obtained from fresh post-mortem skin, which was separated according to the method of Kligman et al. (Arch. Derm. 88, 702, 1963. Preparation of the isolated sheets of the human stratum corneum).

The cumulative amount of prostaglandin permeated into the stratum corneum per unit area at each time point is shown in Fig. 1. The results show that prostaglandin permeated the stratum corneum in a high amount within the first 30 min. These data indicate that an effective amount of the drug can be delivered to the site of action in a simple, safe, efficacious and timely manner, overcoming the deficiencies of systems described in the prior art.

## Claims

1. A delivery device for treatment of erectile dysfunction in a patient, comprising a disk formed from a filmogenic polymer, and having an effective dose of a therapeutic agent selected from the group consisting of: a vasodilator, a smooth muscle relaxant, an antidepressant, a parasympathetic stimulator, a renin-angiotensin system inhibitor, a local anesthetic, an a-blocker, and a calcium channel blocker.

2. A delivery device according to claim 1, wherein the therapeutic agent is a prostaglandin.

3. A delivery agent according to claim 2, wherein the prostaglandin is prostaglandin E1.

4. A delivery agent according to any of claims 1 to 3, wherein the therapeutic agent is misoprostol.

5. A delivery device according to claim 1, wherein the therapeutic agent is selected from the group consisting of: a prostaglandin, a testosterone, a yohimbine, a pentoxifylline, a trazodone, an apomorphine, a phentolamine, a sildenafil, a minoxidil, a misoprostol, a papaverine, a nitroglycerin, a phentolamine, a moxisylyte, a linsidomine, a linear peptide, a cyclic peptide, and a pyridylguanidine compound.

6. A delivery device according to any of claims 1 to 5, further comprising at least an additional therapeutic agent.

7. A delivery device according to any of claims 1 to 6, comprising further at least one additive contained within the disk, wherein the at least one additive is selected from the group consisting of a stabilizer, a solubilizer, an enhancer and a plasticizer.

8. A delivery device according to claim 7, wherein the enhancer is at least one selected from the group consisting of a glycolipid, a non-esterified fatty acid, an aliphatic alcohol, a fatty acid ester of an aliphatic alcohol, a cyclohexanol, a cyclohexanol derivative, a fatty acid ester of glycerol, a glycol, an aliphatic alcohol ether of a glycol, and a surfactant.

9. A delivery device according to any of claims 1 to 8, wherein the filmogenic polymer is selected from the group consisting of a synthetic polymer, a semi-synthetic polymer, and a naturally occurring polymer.

10. A delivery device according to claim 9, wherein the synthetic polymer is polyvinyl pyrrolidone.

11. A delivery device according to claim 9, wherein the naturally occurring polymer is from a plant.

12. A delivery device according to claim 11, wherein the plant polymer is plant protein, preferably a prolamine, and even more preferably a gliadin.

13. A delivery device according to any of claims 7 to 12, wherein the filmogenic polymer is polyvinyl pyrrolidone, the therapeutic agent is prostaglandin E1, the enhancer is Eutanol™ G16S, and the plasticizer is polyethylene glycol 400.

14. A delivery device according to any of claims 7 to 13, wherein the filmogenic material is present in an amount of 5 to 100 %, preferably 70-95 % w/w, the therapeutic agent is present in an amount of 0, 1 to 20 % w/w, the enhancer is present in an amount of 0,01 1 to 15 %, and the plasticizer is present in an amount of 1 to 70 %, each on a dry weight basis.

15. A delivery device according to claim 14, having the filmogenic material present in an amount of 70 to 95 %, the therapeutic agent present in an amount of 1 to 10%, the permeation enhancer present in an amount of 1 to 10 %, and the plasticizer present in an amount of 3 to 50 %.

16. A delivery device according to claim 15, having polyvinyl pyrrolidone present in an amount that is 40 to 45 %, having prostaglandin E1 present in an amount that is 5 to 10 %, having Eutanol™ G16S present in an amount that is 1 to 4 %, and having polyethylene glycol 400 present in an amount that is 40 to 50 %.

17. A delivery device according to any of claims 7 to 15, having a plasticizer in an amount less than 30% on a dry weight basis, and forming a rigid disk.

18. A delivery device according to any of claims 7 to 14, having a plasticizer present in an amount of 30 to 60 % on a dry weight basis, preferably 40-50% and forming a flexible disk.

19. A delivery device according to any of claims 1 to 18, wherein delivery is transdermal.

20. A delivery device according to any of claims 1 to 18, wherein delivery is transmucosal.

21. A delivery device according to any of claims 1 to 20, wherein the effective dose is released into the subject within one hour.

22. Use of a disk formed from a filmogenic polymer, and having an effective dose of a therapeutic agent selected from the group consisting of: a vasodilator, a smooth muscle relaxant, an antidepressant, a parasympathetic stimulator, a renin-angiotensin system inhibitor, a local anesthetic, an a-blocker, and a calcium channel blocker for the manufacture of a delivery device according to any of claims 1 to 21 to be applied to the penile surface during an effective period of time for treating erectile dysfunction.

23. Use according to claim 22, wherein said therapeutic agent is selected from the group consisting of prostaglandin, a testosterone, a yohimbine, a pentoxifylline, a trazodone, an apomorphine, a phentolamine, a sildenafil, a minoxidil, a misoprostol, a papaverine, a nitroglycerin, a phentolamine, a moxisylyte, a linsidomine, a linear peptide, a cyclic peptide and a pyridylguanidine compound.

24. Use according to claim 22 or 23, wherein the therapeutic agent is present in a range of 0,1-15 %, on a dry weight basis.

25. Use according to any of claims 22 to 24, wherein forming the disk further comprises adding a plasticizer.

26. Use according to claim 25, wherein the plasticizer is present in an amount that is less than 30% on a dry weight basis, and delivering the therapeutic agent to the penile surface does not require pre-wetting.

27. Use according to claim 25, wherein the plasticizer is present in an amount that is 30% to 60% on a dry weight basis, and delivering the therapeutic agent to the penile surface has the additional step of pre-wetting the surface.

28. Use according to any of claims 25 to 27, wherein the plasticizer is a polyethylene glycol (PEG).

29. Use according to claim 28, wherein the polyethylene glycol is polyethylene glycol 400.

30. Use according to any of claims 22 to 29, wherein the filmogenic polymer is a synthetic polymer.

31. Use according to claim 30, wherein the synthetic polymer is polyvinyl pyrrolidone.

32. Use according to any of claims 22 to 29, wherein the filmogenic polymer is a plant protein.

33. Use according to claim 32, wherein the plant protein is a prolamine, and more preferably a gliadin.

34. Use according to any of claims 22 to 33, wherein the effective period of time is 5 to 100 minutes.

35. Use according to claim 34, wherein the effective period of time is 30-60 minutes.

36. Use according to any of claims 22 to 35, wherein the penile surface is selected from the group consisting of the shaft and the glans.

37. A method of preparation of a flexible disk for treatment of erectile dysfunction, comprising:
preparing a composition having prostaglandin E1, Eutanol™ G16S, polyvinyl pyrrolidone, and polyethylene glycol 400, the said polyethylene glycol 400 being in an amount of 30 to 60 % on a dry weight basis ; and
forming the composition to have a backing and a release layer.

38. A method according to claim 37, wherein preparing the composition includes adding prostaglandin E1 in an amount that is 5 to 10%, adding Eutanol™ G16S in an amount that is 1 to 5%, adding polyvinyl pyrrolidone in an amount that is 40 to 50%, and adding polyethylene glycol 400 in an amount that is 40 to 50%, each on a dry weight basis.

39. Flexible disk for treatment of erectile dysfunction susceptible to be obtained according to a method of preparation of claim 37 or 38.

40. A method of preparation of a rigid disk for treatment of erectile dysfunction, comprising:
preparing a composition having prostaglandin E1, Eutanol™ G16S, polyvinyl pyrrolidone, and polyethylene glycol 400, the said polyethylene glycol 400 being in an amount less than 30 % on a dry weight basis ; and
forming a disk which does not require a backing or a release layer.

41. Rigid disk for treatment of erectile dysfunction susceptible to be obtained according to a method of preparation of claim 40.

## Patentansprüche

1. Eine Vorrichtung zur Verabreichung einer Behandlung von erektiler Dysfunktion in einem Patienten, bestehend aus einer aus filmogenen Polymer gebildeten Scheibe, welche auch eine wirkungsvolle Dosis eines therapeutischen Wirkstoffes hat, gewählt aus der Gruppe bestehend aus: einem Vasodilatator, einem Relaxator für glatte Muskulatur, einem Antidepressor, einem parasympatischen Stimulator, einem Hemmstoff für das Renin-angiotensin System, einem lokalen Betäubungsmittel, einem A-Blockierer und einem Kalziumkanal Blockierer.

2. Eine Vorrichtung zur Verabreichung nach Anspruch 1, bei der der therapeutische Wirkstoff Prostaglandin ist.

3. Eine Vorrichtung zur Verabreichung nach Anspruch 2, bei dem das Prostaglandin Prostaglandin E1 ist.

4. Eine Vorrichtung zur Verabreichung nach einem der Ansprüche 1 bis 3, bei der der therapeutische Wirkstoff Misoprostol ist.

5. Eine Vorrichtung zur Verabreichung nach Anspruch 1, bei der der therapeutische Wirkstoff aus der Gruppe bestehend aus: einem Prostaglandin, einem Testosteron, einem Yohimbin, einem Pentoxifyllin, einem Trazodon, einem Apomorphin,, einem Phentolamin, einem Sildenafil, einem Minoxidil, einem Misoprostol, einem Papaverin, einem Nitroglycerin, einem Phentolamin, einem Moxisylyt, einem Linsidomin, einem linearen Peptid, einem zyklisch angeordneten Peptid und einer Pyridylguanidin Verbindung, gewählt ist.

6. Eine Vorrichtung zur Verabreichung nach einem der Ansprüche 1 bis 5, welche ferner mindestens einen zusätzlichen therapeutischen Wirkstoff umfasst.

7. Eine Vorrichtung zur Verabreichung nach einem der Ansprüche 1 bis 6, welche ferner mindestens ein in der Scheibe enthaltenes Additiv umfasst, wobei das mindestens eine Additiv aus der Gruppe bestehend aus einem Stabilisator, einem Lösungsmittel, einem Verstärker und einem Weichmacher gewählt ist.

8. Eine Vorrichtung zur Verabreichung nach Anspruch 7, bei der der Verstärker mindestens einer aus der Gruppe bestehend aus einem Glycolipid, einer Fettsäure ohne Ester, einem aliphatischen Alkohol, einem Fettsäure Ester eines aliphatischen Alkohols, einem Cyclohexanol, einem Cyclohexanol Derivat, einem Gycerol Fettsäure Ester, einem Glykol, einem Glykol aliphatischem Alkohol Ether und einem oberflächenaktiven Mittel gewählt ist.

9. Eine Vorrichtung zur Verabreichung nach einem der Ansprüche 1 bis 8, bei der das filmogene Polymer aus der Gruppe bestehend aus einem synthetischen Polymer, einem halb-synthetischen Polymer und einem natürlich auftretenden Polymer gewählt ist.

10. Eine Vorrichtung zur Verabreichung nach Anspruch 9, bei der das synthetische Polymer Polyvinyl Pyrrolidon ist.

11. Eine Vorrichtung zur Verabreichung nach Anspruch 9, bei der das natürlich auftretende Polymer von einer Pflanze stammt.

12. Eine Vorrichtung zur Verabreichung nach Anspruch 11, bei der das pflanzliche Polymer pflanzliches Protein ist, vorzugsweise ein Prolamin, und auf noch bevorzugte Weise ein Gliadin.

13. Eine Vorrichtung zur Verabreichung nach einem der Ansprüche 7 bis 12, bei der das filmogene Polymer Polyvinyl Pyrrolidon ist, der therapeutsiche Wirksotff Prostaglandin E1 ist, der Verstärker Eutanol™ G16S ist und der Weichmacher Poylethylen Glykol 400 ist.

14. Eine Vorrichtung zur Verabreichung nach einem der Ansprüche 7 bis 13, bei der der filmogene Stoff zu einer Menge von 5 bis 100%, vorzugsweise 70-95% w/w vorhanden ist, der therapeutische Wirkstoff zu einer Menge von 0,1 bis 20% w/w vorhanden ist, der Verstärker zu einer Menge von 0,01 bis 15% vorhanden ist, und der Weichmacher zu einer Menge von 1 bis 70% vorhanden ist, jeweils als Trockengewicht ausgedrückt.

15. Eine Vorrichtung zur Verabreichung nach Anspruch 14, wobei der filmogene Stoff zu einer Menge von 70 bis 95% vorhanden ist, der therapeutische Wirkstoff zu einer Menge von 1 bis 10% vorhanden ist, der Permeation Verstärker zu einer Menge von 1 bis 10% vorhanden ist, und der Weichmacher zu einer Menge von 3 bis 50% vorhanden ist.

16. Eine Vorrichtung zur Verabreichung nach Anspruch 15, wobei sie Polyvinyl Pyrrolidon zu einer Menge von 40 bis 45% enthält, Prostaglandin E1 zu einer Menge von 5 bis 10% enthält, Eutanol™ G16S zu einer Menge von 1 bis 4% enthält und Polyethylen Glykol 400 zu einer Menge von 40 bis 50% enthält.

17. Eine Vorrichtung zur Verabreichung nach einem der Ansprüche 7 bis 15, wobei sie einen Weichmacher zu einer Menge von weniger als 30% als Trockengewicht ausgedrückt enthält, und eine steife Scheibe bildet.

18. Eine Vorrichtung zur Verabreichung nach einem der Ansprüche 7 bis 14, wobei sie einen Weichmacher zu einer Menge von 30 bis 60% als Trockengewicht ausgedrückt enthält, vorzugsweise zu 40-50% und eine flexible Scheibe bildet.

19. Eine Vorrichtung zur Verabreichung nach einem der Ansprüche 1 bis 18, bei der die Verabreichung transdermal ist.

20. Eine Vorrichtung zur Verabreichung nach einem der Ansprüche 1 bis 18, bei der die Verabreichung transmucosal ist.

21. Eine Vorrichtung zur Verabreichung nach einem der Ansprüche 1 bis 20, bei der die wirkungsvolle Dosis in den Patienten innerhalb von einer Stunde freigesetzt wird.

22. Verwendung einer aus filmogenen Polymer gebildeten Scheibe und welche eine wirkungsvolle Dosis eines therapeutischen Wirkstoffs enthält, ausgewählt aus der Gruppe bestehend aus: einem Vasodilatator, einem Relaxator für glatte Muskulatur, einem Antidepressor, einem parasympatischen Stimulator, einem Hemmstoff für das Renin-angiotensin System, einem lokalen Betäubungsmittel, einem A-Blockierer und einem Kalziumkanal Blockierer zur Fertigung eine Vorrichtung zu Verabreichung nach einem der Ansprüche 1 bis 21, welche während einem wirkungsvollen Zeitraum auf die Penisoberfläche zur Behandlung erektiler Dysfunktion aufgebracht wird.

23. Verwendung nach Anspruch 22, bei der der besagte therapeutische Wrikstoff aus der Gruppe bestehend aus: einem Prostaglandin, einem Testosteron, einem Yohimbin, einem Pentoxifyllin, einem Trazodon, einem Apomorphin,, einem Phentolamin, einem Sildenafil, einem Minoxidil, einem Misoprostol, einem Papaverin, einem Nitroglycerin, einem Phentolamin, einem Moxisylyt, einem Linsidomin, einem linearen Peptid, einem zyklisch angeordneten Peptid und einer Pyridylguanidin Verbindung gewählt ist.

24. Verwendung nach Anspruch 22 oder 23, bei der der therapeutische Wirkstoff in einem Umfang von 0,1-15% als Trockengewicht ausgedrückt vorhanden ist.

25. Verwendung nach einem der Ansprüche 22 bis 24, bei der die Formung der Scheibe ferner die Zugabe eines Weichmachers umfasst.

26. Verwendung nach Anspruch 25, bei der der Weichmacher zu einer Menge von weniger als 30% als Trockengewicht ausgedrückt vorhanden ist, und die Verabreichung des therapeutischen Wirkstoffes auf die Penisoberfläche kein vorrausgehendes Anfeuchten erfordert.

27. Verwendung nach Anspruch 25, bei der der Weichmacher zu einer Menge von 30% bis 60% als Trockengewicht ausgedrückt vorhanden ist, und die Verabreichung des therapeutischen Wirkstoffes den zusätzlichen Schritt des vorausgehenden Anfeuchten der Öberfläche beinhaltet.

28. Verwendung nach einem der Ansprüche 25 bis 27, bei der der Weichmacher ein Polyethylen Glykol (PEG) ist.

29. Verwendung nach Anspruch 28, bei der das Polyethylen Glykol Polyethylen Glykol 400 ist.

30. Verwendung nach einem der Ansprüche 22 bis 29, bei der das filmogene Polymer ein synthetisches Polymer ist.

31. Verwendung nach Anspruch 30, bei der das synthetische Polymer Polyvinyl Pyrrolidon ist.

32. Verwendung nach einem der Ansprüche 22 bis 29, bei der das filmogene Polymer ein pflanzliches Protein ist.

33. Verwendung nach Anspruch 32, bei der das pflanzliche Protein ein Prolamin ist und vorzugsweise ein Gliadin.

34. Verwendung nach einem der Ansprüche 22 bis 33, bei der der wirkungsvolle Zeitraum von 5 bis 100 Minuten ist.

35. Verwendung nach Anspruch 34, bei der der wirkungsvolle Zeitraum von 30 bis 60 Minuten ist.

36. Verwendung nach einem der Ansprüche 22 bis 35, bei der die Penisoberfläche aus der Gruppe bestehend aus Corpus und Glans gewählt ist.

37. Methode zur Zubereitung einer flexiblen Scheibe zur Behandlung von erektiler Dysfunktion bestehend aus:
der Zugabe eines Präparats mit Prostaglandin E1, Eutanol™ G16S, Polyvinyl Pyrrolidon, und Polyethylen Glykol 400, wobei das besagte Polyethylen Glykol 400 zu einer Menge von 30 bis 60% als Trockengewicht ausgedrückt enthalten ist; und
die Formung des Präparats, um eine Schicht zur Stützung und zur Freisetzung zu haben.

38. Methode nach Anspruch 37, bei der die Zubereitung des Präparats die Zugabe von Prostaglandin E1 zu einer Mange von 5 bis 10%, die Zugabe von Eutanol™ G16S zu einer Menge von 1 bis 5%, die Zugabe von Polyvinyl Pyrrolidon zu einer Menge von 40 bis 50%, die Zugabe von Polyethylen Glykol 400 zu einer Menge von 40 bis 50% jeweils als Trockengewicht ausgedrückt, umfasst.

39. Flexible Scheibe zur Behandlung von erektiler Dysfunktion, welche gemä einer Zubereitungsmethode der Ansprüche 37 oder 38 gewonnen werden kann.

40. Methode zur Zubereitung einer steifen Scheibe zur Behandlung von erektiler Dysfunktion, bestehend aus:
der Zugabe eines Präparats mit Prostaglandin E1, Eutanol™ G16S, Polyvinyl Pyrrolidon, und Polyethylen Glykol 400, wobei das besagte Polyethylen Glykol 400 zu einer Menge von weniger als 30% als Trockengewicht ausgedrückt enthalten ist; und
die Formung einer Scheibe, welche keine Schicht zur Stützung oder zur Freisetzung benötigt.

41. Steife Scheibe zur Behandlung von erektiler Dysfunktion, welche gemä der Zubereitungsmethode des Anspruchs 40 gewonnen werden kann.

## Revendications

1. Dispositif de délivrance destiné au traitement d'un dysfonctionnement érectile chez un patient, comprenant un disque constitué d'un polymère filmogène et comportant une dose efficace d'un agent thérapeutique choisi dans le groupe constitué des vasodilatateurs, dépresseurs des fibres lisses, antidépresseurs, activateurs du système parasympathique, inhibiteurs du système rénine-angiotensine, anesthésiques locaux, alpha-bloquants et inhibiteurs calciques.

2. Dispositif de délivrance selon la revendication 1, dans lequel l'agent thérapeutique est une prostaglandine.

3. Agent de délivrance selon la revendication 2, dans lequel la prostaglandine est la prostaglandine E1.

4. Agent de délivrance selon l'une quelconque des revendications 1 à 3, dans lequel l'agent thérapeutique est le misoprostol.

5. Dispositif de délivrance selon la revendication 1, dans lequel l'agent thérapeutique est choisi dans le groupe constitué des prostaglandines, de la testostérone, de la yohimbine, de la pentoxifylline, de la tradozone, de l'apomorphine, de la phentolamine, du sildénafil, du minoxidil, du misoprostol, de la papavérine, de la nitroglycérine, de la phentolamine, du moxisylyte, de la linsidomine, des peptides linéaires, des peptides cycliques et des composés à base de pyridylguanidine.

6. Dispositif de délivrance selon l'une quelconque des revendications 1 à 5, comprenant, en outre, au moins un agent thérapeutique supplémentaire.

7. Dispositif de délivrance selon l'une quelconque des revendications 1 à 6, comprenant, en outre, au moins un additif contenu dans le disque, le au moins un additif étant choisi dans le groupe constitué des stabilisants, solubilisants, activateurs et plastifiants.

8. Dispositif de délivrance selon la revendication 7, dans lequel figure au moins un activateur choisi dans le groupe constitué des glycolipides, des acides gras non estérifiés, des alcools aliphatiques, des esters d'acide gras et d'alcool aliphatique, du cyclohexanol, des dérivés du cyclohexanol, des esters d'acide gras et de glycérol, des glycols, des éthers d'alcool aliphatique et de glycol et des surfactants.

9. Dispositif de délivrance selon l'une quelconque des revendications 1 à 8, dans lequel le polymère filmogène est choisi dans le groupe constitué des polymères synthétiques, des polymères semi-synthétiques et des polymères naturels.

10. Dispositif de délivrance selon la revendication 9, dans lequel le polymère synthétique est la polyvinylpyrrolidone.

11. Dispositif de délivrance selon la revendication 9, dans lequel le polymère naturel est d'origine végétale.

12. Dispositif de délivrance selon la revendication 11, dans lequel le polymère végétal est une protéine végétale, de préférence une prolamine et, de façon encore préférée, une gliadine.

13. Dispositif de délivrance selon l'une quelconque des revendications 7 à 12, dans lequel le polymère filmogène est la polyvinylpyrrolidone, l'agent thérapeutique est la prostaglandine E1, l'activateur est l'Eutanol™ G16S et le plastifiant est le polyéthylèneglycol 400.

14. Dispositif de délivrance selon l'une quelconque des revendications 7 à 13, dans lequel le matériau filmogène est présent à hauteur de 5 à 100 % et, de préférence, de 70 à 95 % poids/poids, l'agent thérapeutique est présent à hauteur de 0,1 à 20 % poids/poids, l'activateur est présent à hauteur de 0,01 à 15 % et le plastifiant est présent à hauteur de 1 à 70 %, chacun sur la base du poids sec.

15. Dispositif de délivrance selon la revendication 14, dans lequel le matériau filmogène est présent à hauteur de 70 à 95 %, l'agent thérapeutique est présent à hauteur de 1 à 10 %, l'activateur de perméation est présent à hauteur de 1 à 10 % et le plastifiant est présent à hauteur de 3 à 50 %.

16. Dispositif de délivrance selon la revendication 15, dans lequel la polyvinylpyrrolidone est présente à hauteur de 40 à 45 %, la prostaglandine E1 est présente à hauteur de 5 à 10 %, l'Eutanol™ G16S est présent à hauteur de 1 à 4 % et le polyéthylèneglycol 400 est présent à hauteur de 40 à 50 %.

17. Dispositif de délivrance selon l'une quelconque des revendications 7 à 15, dans lequel un plastifiant est présent à hauteur de moins de 30 % sur la base du poids sec, ledit dispositif formant un disque rigide.

18. Dispositif de délivrance selon l'une quelconque des revendications 7 à 14, dans lequel un plastifiant est présent à hauteur de 30 à 60 % sur la base du poids sec et, de préférence, à hauteur de 40 à 50 %, ledit dispositif formant un disque souple.

19. Dispositif de délivrance selon l'une quelconque des revendications 1 à 18, dans lequel la délivrance se fait par voie transdermique.

20. Dispositif de délivrance selon l'une quelconque des revendications 1 à 18, dans lequel la délivrance se fait par voie transmuqueuse.

21. Dispositif de délivrance selon l'une quelconque des revendications 1 à 20, dans lequel la libération, chez le sujet, de la dose efficace est effectuée en une heure.

22. Utilisation d'un disque constitué d'un polymère filmogène et comportant une dose efficace d'un agent thérapeutique choisi dans le groupe constitué des vasodilatateurs, dépresseurs des fibres lisses, antidépresseurs, activateurs du système parasympathique, inhibiteurs du système rénine-angiotensine, anesthésiques locaux, alpha-bloquants et inhibiteurs calciques, pour la fabrication d'un dispositif de délivrance, selon l'une quelconque des revendications 1 à 21, destiné à être appliqué sur la surface du pénis pendant une durée efficace en vue de traiter un dysfonctionnement érectile.

23. Utilisation selon la revendication 22, dans laquelle ledit agent thérapeutique est choisi dans le groupe constitué des prostaglandines, de la testostérone, de la yohimbine, de la pentoxifylline, de la tradozone, de l'apomorphine, de la phentolamine, du sildénafil, du minoxidil, du misoprostol, de la papavérine, de la nitroglycérine, de la phentolamine, du moxisylyte, de la linsidomine, des peptides linéaires, des peptides cycliques et des composés à base de pyridylguanidine.

24. Utilisation selon les revendications 22 ou 23, dans laquelle l'agent thérapeutique est présent à hauteur de 0,1 à 15 % sur la base du poids sec.

25. Utilisation selon l'une quelconque des revendications 22 à 24, dans laquelle la fabrication du disque implique, en outre, l'addition d'un plastifiant.

26. Utilisation selon la revendication 25, dans laquelle le plastifiant est présent à hauteur de moins de 30 % sur la base du poids sec, et dans laquelle la délivrance de l'agent thérapeutique au niveau de la surface du pénis n'exige pas d'humidification préalable.

27. Utilisation selon la revendication 25, dans laquelle le plastifiant est présent à hauteur de 30 à 60 % sur la base du poids sec, et dans laquelle la délivrance de l'agent thérapeutique au niveau de la surface du pénis implique une étape supplémentaire consistant à pré-humidifier ladite surface.

28. Utilisation selon l'une quelconque des revendications 25 à 27, dans laquelle le plastifiant est un polyéthylèneglycol (PEG).

29. Utilisation selon la revendication 28, dans laquelle le polyéthylèneglycol est le polyéthylèneglycol 400.

30. Utilisation selon l'une quelconque des revendications 22 à 29, dans laquelle le polymère filmogène est un polymère synthétique.

31. Utilisation selon la revendication 30, dans laquelle le polymère synthétique est la polyvinylpyrrolidone.

32. Utilisation selon l'une quelconque des revendications 22 à 29, dans laquelle le polymère filmogène est une protéine végétale.

33. Utilisation selon la revendication 32, dans laquelle la protéine végétale est une prolamine et, de préférence, une gliadine.

34. Utilisation selon l'une quelconque des revendications 22 à 33, dans laquelle la durée efficace est comprise entre 5 et 100 minutes.

35. Utilisation selon la revendication 34, dans laquelle la durée efficace est comprise entre 30 et 60 minutes.

36. Utilisation selon l'une quelconque des revendications 22 à 35, dans laquelle la surface du pénis est choisie dans le groupe constitué du corps de la verge et du gland.

37. Procédé de préparation d'un disque souple destiné au traitement d'un dysfonctionnement érectile, comprenant :
la préparation d'une composition comportant de la prostaglandine E1, de l'Eutanol™ G16S, de la polyvinylpyrrolidone et du polyéthylèneglycol 400, ledit polyéthylèneglycol 400 étant présent à hauteur de 30 à 60 % sur la base du poids sec ; et
le façonnage de la composition de façon à ce qu'elle comporte une couche de support et une couche de délivrance.

38. Procédé selon la revendication 37, dans lequel la préparation de la composition comprend l'adjonction de prostaglandine E1 à hauteur de 5 à 10 %, l'adjonction d'Eutanol™ G16S à hauteur de 1 à 5 %, l'adjonction de polyvinylpyrrolidone à hauteur de 40 à 50 % et l'adjonction de polyéthylèneglycol 400 à hauteur de 40 à 50 %, chacun sur la base du poids sec.

39. Disque souple destiné au traitement d'un dysfonctionnement érectile, susceptible d'être obtenu selon le procédé de préparation des revendications 37 ou 38.

40. Procédé de préparation d'un disque rigide destiné au traitement d'un dysfonctionnement érectile, comprenant :
la préparation d'une composition comportant de la prostaglandine E1, de l'Eutanol™ G16S, de la polyvinylpyrrolidone et du polyéthylèneglycol 400, ledit polyéthylèneglycol 400 étant présent à hauteur de moins de 30 % sur la base du poids sec ; et
le façonnage d'un disque ne nécessitant pas de couche de support ni de couche de délivrance.

41. Disque rigide destiné au traitement d'un dysfonctionnement érectile, susceptible d'être obtenu selon le procédé de préparation de la revendication 40.
